# FASCICULE DE BREVET EUROPEEN

(11) **EP 2 663 286 B1**
(45) Date de publication et mention de la délivrance du brevet: **10.04.2019**
(21) Numéro de dépôt: 12705316.3
(22) Date de dépôt: 10.01.2012
(51) Int. Cl.: A61K 9/08, A61K 9/10, A61K 9/14, A61K 31/198, A61P 35/00, A61P 35/02, A61P 35/04

(54) **ACTION PREVENTIVE DE LA CITRULLINE SUR LE DEVELOPPEMENT SPONTANE DES TUMEURS**
PRÄVENTIVE WIRKUNG VON CITRULLIN AUF DIE FREIE ENTWICKLUNG VON TUMOREN
PREVENTIVE EFFECT OF CITRULLINE ON THE SPONTANEOUS DEVELOPMENT OF TUMORS

(30) Priorité: 14.01.2011 FR 1150311
(43) Date de publication de la demande: 20.11.2013
(73) Titulaire: Cynober, Luc, 92330 Sceaux (FR); Baracos, Vickie, Spruce Grove, AB T7Y 1C6 (CA)
(72) Inventeur: MOINARD, Christophe, F-92340 Bourg-la-reine (FR); LE PLENIER, Servane, F-78650 Beynes (FR); RAYNAUD-SIMON, Agathe, F-94300 Vincennes (FR); BARACOS, Vickie, Spruce Grove Alberta T7Y 1C6 (CA); CYNOBER, Luc, F-92330 Sceaux (FR)
(74) Mandataire: Bourgouin, André
(86) Numéro de dépôt international: PCT/FR2012/050068
(87) Numéro de publication internationale: WO 2012/095607

(56) Documents cités:
- WO-A1-2009/070378
- WO-A2-2007/114903
- CN-A- 101 822 622
- DE-A1- 19 533 330
- US-A- 4 988 724
- FUJITA H ET AL: "ANTI TUMOR ACTIVITY OF A NEW AMINO-ACID DERIVATIVE N-8 N-9 BIS BUTYLOXYCARBONYLAMINOMETHYL-L CITRULLINE", GANN, vol. 69, no. 1, 1978, pages 99-101, XP002656484, ISSN: 0016-450X
- DATABASE WPI Week 199727 Thomson Scientific, London, GB; AN 1997-296834 XP002656485, & SU 1 834 006 A1 (AS USSR URALS SECT CHEM INST) 27 octobre 1996 (1996-10-27)
- DATABASE WPI Week 201106 Thomson Scientific, London, GB; AN 2010-M44738 XP002656486, & CN 101 822 622 A (YAO C) 8 septembre 2010 (2010-09-08)

## Description

La présente invention a pour objet une nouvelle utilisation de la citrulline.

La citrulline (ou acide 2-amino-5-(carbamoyl amino)pentanoïque) est un acide α-aminé et a été isolée pour la première fois à partir de la pastèque. La citrulline est un acide aminé non essentiel que l'organisme produit (au niveau intestinal) à partir d'autres nutriments. Par exemple, la citrulline joue un rôle particulièrement important, avec l'ornithine et l'arginine, dans le cycle de l'urée. Enfin, la citrulline joue un rôle majeur dans l'homéostasie de l'arginine et du monoxyde d'azote radicalaire.

Certaines applications pharmaceutiques de la citrulline ont déjà été décrites.

Ainsi, la demande de brevet européen EP 1495755 concerne l'utilisation de la citrulline pour la préparation d'un médicament destiné au traitement de pathologies liées à une insuffisance intestinale. Plus particulièrement, les pathologies mentionnées dans cette demande sont les suivantes : le syndrome du grêle court faisant suite à une résection intestinale, la maladie coeliaque, les maladies inflammatoires chroniques de l'intestin, l'insuffisance intestinale liée au vieillissement et l'insuffisance intestinale liée à une irradiation. La demande de brevet européen EP1755582 décrit notamment l'utilisation de l'association d'au moins une statine, avec la citrulline pour la préparation d'un médicament destiné à la prévention ou au traitement de l'athérosclérose, notamment de l'athérosclérose primaire ou secondaire, de l'hypertension, du diabète, ou des maladies neurodégénératives, notamment la maladie d'Alzheimer. Le brevet FR2937550 décrit l'utilisation de la citrulline dans le traitement et la prévention du syndrome d'ischémie-reperfusion. La demande US 2009/02911877 décrit l'utilisation de la citrulline dans le traitement de la satiété et de la dyspepsie notamment chez les sujets âgés ou chez les patients cancéreux. La demande WO 2008/049984 décrit l'utilisation de la citrulline dans le traitement des états de dénutrition chez les personnes atteintes d'un cancer. La demande WO 2007/114903 décrit l'utilisation de la L-citrulline pour le traitement des troubles digestifs comme la notion de satiété et la dyspepsie, notamment chez des malades atteints de cancers. Le document DE19533330 est également pertinent.

Il est désormais admis par tous que l'incidence de la plupart des cancers augmente avec l'avance en âge. Ainsi en France, 5% des plus de 50 ans déclarent être atteints par un cancer. Si le cancer du sein, le cancer du côlon, etc. sont des maladies qui touchent d'abord les personnes âgées, c'est parce qu'un ensemble de mutations et d'événements concourt à leur formation, par exemple, des modifications de l'imprégnation hormonale ou l'exposition répétée à des agents carcinogènes d'origine alimentaire ou environnementaux.

En effet, bien que des facteurs génétiques soient profondément impliqués dans l'apparition des cancers, les facteurs environnementaux, en particulier nutritionnels, jouent également un rôle important. L'apparition des cancers est notamment répandue dans les pays développés et est liée à l'utilisation des pesticides, des insecticides, à leurs quantités résiduelles dans les aliments, à la consommation d'additifs tels que des agents conservateurs et des agents colorants, à la pollution de l'eau, du sol et de l'air, à l'obésité provoquée par des habitudes diététiques riches en graisses saturées, à la prolongation de la durée de vie qui favorise l'exposition répétée à des agents carcinogènes et aux altérations du cycle cellulaire.

Les divers procédés de traitement de cancer sont principalement le traitement chirurgical, la chimiothérapie, l'immunothérapie et la radiothérapie. Le procédé chirurgical de traitement est efficace dans les stades précoces, mais conduit à l'élimination de l'organe malade, ce qui peut provoquer des pathologies secondaires néfastes, et n'empêche pas toujours la propagation du cancer à d'autres organes. La radiothérapie est avantageuse puisqu'elle permet un traitement plus sélectif de l'organe malade, mais n'empêche pas non plus la propagation du cancer à d'autres organes. La chimiothérapie est connue pour agir non seulement sur les cellules cancéreuses mais également sur les cellules normales d'un patient provoquant des effets secondaires parfois invalidant. Ainsi, les traitements anticancéreux vont avoir un impact majeur sur la sphère digestive (qui va se traduire par l'apparition de nausées, de vomissements et le développement d'une anorexie) et par une cachexie qui met enjeu le pronostic vital.

Ainsi, malgré les grands succès déjà obtenus dans le combat contre le cancer, la prévention et le traitement de cette maladie ne sont généralement pas satisfaisants. Par conséquent, il reste nécessaire de mettre au point des moyens plus efficaces du traitement des cancers et surtout, dans la mesure du possible, d'essayer de les prévenir. En effet, l'ensemble des stratégies anticancéreuses développées a des effets secondaires qui sont très invalidants et qui peuvent engager le pronostic vital (à titre d'exemple, les inhibiteurs de mTOR vont amplifier le phénomène de cachexie).

Le brevet US 4988724 décrit des solutions parentérales susceptibles de prévenir la stimulation de la croissance des tumeurs chez des patients cancéreux, lesdites solutions contenant un mélange d'acides aminés essentiels, d'ornithine et de citrulline, d'acides aminés non-essentiels et étant pratiquement dépourvue d'arginine. La composition de ces solutions est basée sur la découverte que l'arginine, précurseur de polyamines, contenue dans les solutions parentérales couramment utilisées chez des malades cancéreux, serait responsable de la croissance des tumeurs. Par conséquent, les inventeurs proposent de remplacer l'arginine par l'ornithine. Dans ces compositions, la citrulline est utilisée pour remplacer en totalité ou en partie l'ornithine en tant que substrat du cycle de l'urée.

Les inventeurs ont découvert de manière surprenante que la citrulline administrée à long terme chez des mammifères sains âgés, réduit la mortalité et diminue l'incidence d'apparition de tumeurs cancéreuses.

Ainsi, un des buts de la présente invention est de fournir un moyen de prévenir ou de traiter les cancers chez le mammifère sain âgé.

La présente invention a en conséquence pour objet l'utilisation de la L-citrulline, ou un sel nutraceutiquement ou pharmaceutiquement acceptable de celle-ci, pour son utilisation dans la prévention du cancer selon la revendication 1 chez l'homme à partir de 70 ans et chez l'animal âgé.

Au sens de la présente invention, on entend par animal âgé, 10 ans chez le chien, 13 ans chez le chat, 20 mois chez les rongeurs et 1 an chez les poissons.

Au titre de la présente invention, on désigne notamment par "sel pharmaceutiquement acceptable" des sels de citrulline tels que le malate de citrulline, l'a-cétoglutarate de citrulline, le citrate de citrulline ou l'a-cétoisocaproate de citrulline.

Au sens de la présente invention on entend par L-citrulline le produit disponible dans le commerce, notamment fourni par Sigma-Aldrich, Biocodex ou Kyowa Hakko ou le produit naturel issu de végétaux, notamment de la pastèque (*Citrullus lanatus*) sous forme notamment de jus, de pulpe ou d'extrait.

Dans un mode de réalisation avantageux de l'invention, la L-citrulline peut se présenter sous forme d'une composition alimentaire en association avec un excipient nutraceutiquement acceptable ou sous forme d'une composition pharmaceutique en association avec un excipient pharmaceutiquement acceptable.

Conformément à l'invention, le cancer est choisi dans le groupe comprenant : les cancers de la tête ou du cou, du poumon, de la plèvre, du foie, des reins, du pancréas ou du système immunitaire (leucémies).

Dans un mode de réalisation avantageux de l'invention, la L-citrulline peut être associée à un composé choisi dans le groupe comprenant la glutamine, les acides gras polyinsaturés (API), les antioxydants comme par exemple les vitamines C, E et le resvératrol,), des micronutriments le zinc, le sélénium ou des anticancéreux (comme par exemple les sels de platine, le 5-fluorouracile ou le méthotrexate) des algues, des nutriments tels que la curcumine, le glucosinolate ou les polyphénols.

Le dosage de la citrulline dépend particulièrement du mode d'administration, et est aisément déterminé par l'homme de métier. Par exemple, sans pour autant être limitatif, les compositions telles qu'utilisées dans l'invention sont susceptibles d'être administrées par voie orale à une dose de 5 à 20g/jour pendant une durée de 1 jour à 10 ans, avantageusement de 1 à 5 ans, par voie parentérale à une dose de 1 à 10 g pendant une durée de 1 mois à 10 ans, notamment dans le cadred'une nutrition au long cours à domicile, avantageusement de 6 mois à 5 ans (on peut utiliser les mêmes doses par voie orale, entérale ou parentérale, avec des modalités d'administration différentes).

Les compositions utilisées selon l'invention peuvent se présenter sous toute forme adaptée à une administration orale, sous-cutanée, intra-veineuse ou entérale en particulier ; elles peuvent se présenter sous forme sèche, sous forme de solution aqueuse ou sous forme d'émulsion.

La présente invention a également pour objet l'utilisation de la L-citrulline ou d'un sel pharmaceutiquement acceptable de celle-ci pour la préparation d'un médicament destiné à la prévention du cancer selon la revendication 1 chez l'homme de plus de 70 ans et l'animal âgé.

La présente invention a également pour objet l'utilisation de la L-citrulline ou d'un sel pharmaceutiquement acceptable de celle-ci pour la préparation d'une composition destinée à la prévention des cancers selon la revendication 1 chez l'homme et l'animal.

La présente invention a également pour objet l'utilisation de la L-citrulline ou d'un sel nutraceutiquement acceptable de celle-ci pour la préparation d'un complément alimentaire ou d'un produit diététique (ADDFMS) destiné à la prévention des cancers selon la revendication 1 chez l'Homme et l'animal.

Au sens de la présente invention, on entend par ADDFMS des aliments destinés à répondre aux besoins nutritionnels de patients dont le métabolisme est perturbé et qui ne peuvent assimiler correctement les nutriments issus d'une alimentation traditionnelle.

La présente invention a également pour objet une méthode permettant la prévention des cancers selon la revendication 1 chez l'homme et l'animal comprenant l'administration au dit mammifère d'une quantité thérapeutiquement efficace de L-citrulline.

L'invention est illustrée par l'exemple qui suit.

### Effet à long-terme de la citrulline chez le rat sain âgé

### 1. Matériel et méthodes

Trente-neuf rats mâles Sprague-Dawley âgés de 20 mois et exempts de tumeurs (Charles River, L'Arbresle, France) sont utilisés. Ils sont élevés individuellement dans des cages, à une température de 20-23°C sous une alternance 12:12-h de cycle jour-nuit. Ils ont libre accès à l'eau. Pendant la période d'acclimatation, tous les rats sont nourris avec un régime standard (59 % de glucides, 3 % de lipides, 17 % de protéines et 21 % de fibres) ad libitum (UAR de 0,4 Dietex, Villemoisson-sur-Orge, France) pendant 2 semaines. Les soins donnés aux animaux sont en accord avec la réglementation française de protection des animaux utilisés à des fins expérimentales et scientifiques (D 2001-486) et avec la réglementation de la Communauté européenne (Journal Officiel de la Communauté européenne, L538 12:18:1986).

Après la période d'acclimatation, les rats sont divisés de manière aléatoire en 2 groupes.

Dans le groupe "citrulline" (CIT, n=19), les rats sont nourris *ad libitum* pendant 12 semaines avec un supplément à base de citrulline (1 g.kg⁻¹.jour⁻¹ de citrulline) mélangé à leur régime standard. La dose de citrulline est calculée par extrapolation des doses utilisées chez l'Homme, en tenant compte du fait que la vitesse de métabolisation et les exigences en azote sont 10 fois plus élevées chez le rongeur que celles mesurées chez l'Homme et est très proche de la dose d'arginine administrée à des rats pendant 12 semaines par Jobgen W. et al. (J Nutr 2009;139:230-7). Cette dose correspond à environ 9% de l'apport azoté soit environ 7g/jour chez le volontaire sain adulte.

Dans le groupe "acides aminés non essentiels" (NEAA, n=20), les rats sont nourris *ad libitum* pendant 12 semaines avec une alimentation standard rendue isoazotée par rapport au groupe citrulline par addition d'acides aminés non essentiels : histidine, serine, alanine and glycine en rapport équimolaire.

Les rats sont euthanasiés à l'état post-absorptif après 12 semaines de traitement.

Les études statistiques sont réalisées par le logiciel Statview.®

### 2. Résultats

### Mortalité

La mortalité sur 12 semaines est de 20 % (4/20) dans le groupe NEAA (ce qui est en accord avec les données de la littérature pour cette espèce et à cet âge) alors qu'aucune mortalité n'est observée dans le groupe CIT (0/19) (NEAA vs CIT, p<0,05)

### Incidence des tumeurs

Après euthanasie, l'incidence des tumeurs est évaluée dans les deux groupes. Dans le groupe NEAA 44 % des rats (7/16) présentent des tumeurs, valeurs en accord avec ce qui est décrit dans la littérature. En revanche, dans le groupe CIT aucun rat ne présente de tumeurs (0/19) (NEAA vs CIT, p<0.05).

### Poids corporel

La supplémentation en citrulline n'affecte pas le poids corporel comme le montre les résultats rassemblés dans le tableau qui suit :

| | NEAA | CIT |
|---|---|---|
| Poids corporel initial | 711 ± 21 | 669 ± 16* |
| Poids corporel final | 658 ± 15 | 630 ± 13** |

| | | |
|---|---|---|
| *NEAA vs CIT p= N.S **NEAA vs CIT p = N.S | | |

### Taux de polyamines

La supplémentation en citrulline induit une forte augmentation d'une polyamine la pytrescine comme le montre le tableau ci-dessous :

| | Groupe citrulline (n=19) | Groupe AANE (n=16) | p |
|---|---|---|---|
| Putrescine foie | 17,1 ± 14,5 | 7,8 ± 5,1 | 0,034 |
| Spermidine foie | 372 ± 90 | 402 ± 119 | NS |
| Spermine foie | 557 ± 245 | 557 ± 353 | NS |
| Putrescine jéjunum | 82,7 ± 36 | 82,8 ± 44,9 | NS |
| Spermidine jéjunum | 478 ± 200 | 429 ± 131 | NS |
| Spermine jéjunum | 297 ± 88 | 293 ± 74 | NS |
| Putrescine iléon | 79,8 ± 25 | 59,3 ± 34,6 | 0,028 |
| Spermidine iléon | 374 ± 70 | 326 ± 96 | NS |
| Spermine iléon | 203 ± 43 | 182 ± 48 | NS |

Ces résultats indiquent clairement que, malgré l'augmentation de la teneur en putrescine, observée après administration de citrulline, de façon surprenante, l'administration de citrulline diminue la mortalité et l'incidence des tumeurs chez les rats âgés alors que les polyamines sont connues pour favoriser la croissance et la prolifération tumorale.

## Revendications

1. L-citrulline ou un sel nutraceutiquement ou pharmaceutiquement acceptable de celle-ci pour son utilisation pour prévenir les cancers chez l'homme sain à partir de 70 ans et chez l'animal sain âgé, dans laquelle le cancer est choisi dans le groupe comprenant: les cancers de la tête ou du cou, du poumon, de la plèvre, du foie, des reins, du pancréas ou du système immunitaire (leucémies) et dans laquelle la composition est administrée par voie orale ou entérale.

2. L-citrulline pour son utilisation selon la revendication 1 **caractérisée en ce qu'**elle se présente sous forme d'une composition alimentaire en association avec un excipient nutraceutiquement acceptable ou sous forme d'une composition pharmaceutique en association avec un excipient pharmaceutiquement acceptable.

3. L-citrulline pour son utilisation selon l'une quelconque des revendications 1 à 2 **caractérisée en ce qu'**elle est associée à un composé choisi dans le groupe comprenant la glutamine, les acides gras polyinsaturés, les antioxydants, des micronutriments et les anticancéreux

4. L-citrulline pour son utilisation selon l'une quelconque des revendications 1 à 3 **caractérisée en ce que** les compositions ont une posologie permettant l'administration d'une dose de 5 à 20g/jour pendant une durée de 1 jour à 10 ans, avantageusement de 1 à 5 ans.

5. L-citrulline pour son utilisation selon l'une quelconque des revendications 1 à 4 **caractérisée en ce que** la composition se présente sous forme sèche, sous forme de solution aqueuse ou sous forme d'émulsion.

## Patentansprüche

1. L-Citrullin oder ein nutrazeutisch oder pharmazeutisch annehmbares Salz davon zur Verwendung bei der Prävention von Krebs beim gesunden Menschen ab 70 Jahren und beim älteren gesunden Tier, wobei der Krebs ausgewählt ist aus der Gruppe umfassend: Krebs des Kopfs oder des Halses, der Lunge, der Pleura, der Leber, der Nieren, der Pankreas oder des Immunsystems (Leukämien), und wobei die Zusammensetzung auf oralem oder enteralem Weg verabreicht wird.

2. L-Citrullin zur Verwendung nach Anspruch 1, **dadurch gekennzeichnet, dass** dieses in der Form einer Nahrungsmittelzusammensetzung in Assoziation mit einem nutrazeutisch annehmbaren Exzipienten oder in der Form einer pharmazeutischen Zusammensetzung in Assoziation mit einem pharmazeutisch annehmbaren Exzipienten dargereicht wird.

3. L-Citrullin zur Verwendung nach einem der Ansprüche 1 bis 2, **dadurch gekennzeichnet, dass** dieses mit einer Verbindung assoziiert ist, ausgewählt aus der Gruppe umfassend Glutamin, polyungesättigte Fettsäuren, Antioxidantien, Mikronährstoffe und Antikrebsmittel.

4. L-Citrullin zur Verwendung nach einem der Ansprüche 1 bis 3, **dadurch gekennzeichnet, dass** die Zusammensetzungen eine Dosierung aufweisen, welche die Verabreichung einer Dosis von 5 bis 20 g/Tag während einer Dauer von 1 Tag bis 10 Jahren, vorteilhaft von 1 Jahr bis 5 Jahren, gestattet.

5. L-Citrullin zur Verwendung nach einem der Ansprüche 1 bis 4, **dadurch gekennzeichnet, dass** die Zusammensetzung in trockener Form, in der Form einer wässrigen Lösung oder in der Form einer Emulsion dargereicht wird.

## Claims

1. L-citrulline or a nutraceutically or pharmaceutically acceptable salt thereof for use in preventing cancers in healthy humans from 70 years of age and in older healthy animals, wherein the cancer is selected from the group consisting of: cancers of the head or neck, lung, pleura, liver, kidney, pancreas or immune system (leukaemias) and wherein the composition is administered orally or enteral.

2. L-citrulline for use according to claim 1 **characterized in that** it is in the form of a food composition in combination with a nutraceutically acceptable excipient or in the form of a pharmaceutical composition in combination with a pharmaceutically acceptable excipient.

3. L-citrulline for use according to any of claims 1 to 2 **characterized in that** it is associated with a compound selected from the group consisting of glutamine, polyunsaturated fatty acids, antioxidants, micronutrients and anticancer agents.

4. L-citrulline for use according to any of claims 1 to 3 **characterized in that** the compositions have a dosage allowing the administration of a dose of 5 to 20 g/day for a period of time from day to 10 years, preferably from 1 to 5 years.

5. L-citrulline for use according to any of claims 1 to 4, **characterized in that** the composition is in dry form, in the form of an aqueous solution or in the form of an emulsion.
